(19) Europäisches Patentamt
European Patent Office
Office européen des brevets

(11) Veröffentlichungsnummer : **0 288 493 B1**

(12) # EUROPÄISCHE PATENTSCHRIFT

(45) Veröffentlichungstag der Patentschrift : **29.05.91 Patentblatt 91/22**

(51) Int. Cl.$^5$ : **C07D 207/416,** A01N 43/36

(21) Anmeldenummer : **87906419.4**

(22) Anmeldetag : **01.10.87**

(86) Internationale Anmeldenummer : **PCT/EP87/00563**

(87) Internationale Veröffentlichungsnummer : **WO 88/02747 21.04.88 Gazette 88/09**

(54) **3-HALOGENSUCCINIMIDE.**

(30) Priorität : **16.10.86 DE 3635282**

(43) Veröffentlichungstag der Anmeldung : **02.11.88 Patentblatt 88/44**

(45) Bekanntmachung des Hinweises auf die Patenterteilung : **29.05.91 Patentblatt 91/22**

(84) Benannte Vertragsstaaten : **AT BE CH DE FR GB IT LI LU NL SE**

(56) Entgegenhaltungen :
**CH-A- 330 820**
**DE-A- 2 012 656**

(73) Patentinhaber : **DOWELANCO**
**9002 Purdue Road**
**Indianapolis, Indiana 46268 (US)**

(72) Erfinder : **HÄBERLE, Norman**
**Willibaldstrasse 51 a**
**W-8000 München 21 (DE)**
Erfinder : **REUTTER, Anneliese**
**Hirschenweg 10**
**W-8011 Eglharting (DE)**

(74) Vertreter : **Huber, Bernhard, Dipl.-Chem. et al**
**Patentanwälte H. Weickmann, Dr. K. Fincke F.A. Weickmann, B. Huber Dr. H. Liska, Dr. J. Prechtel Möhlstrasse 22 Postfach 860 820**
**W-8000 München 86 (DE)**

**EP 0 288 493 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft gewisse N-substituierte 3-Halogensuccinimide, ein Verfahren zu ihrer Herstellung sowie die Verwendung dieser Verbindungen aus fungizide Wirkstoffe.

In DE-AS 20 12 656 und in der JP Kokai 75 129 743 sind einige N-substituierte 3-Halogensuccinimide als fungizide Wirkstoffe beschrieben. In J. Org. Chem. Vol. 37, Nr. 25, (1972) wird die Herstellung von α-Chlor-N-phenylsuccinimid beschrieben.

Aufgabe der Erfindung war es, neue Substanzen mit hoher fungizider Wirksamkeit herzustellen. Eine weitere Aufgabe der vorliegenden Erfindung war es, fungizid wirksame Substanzen mit breitem Wirksamkeitsspektrum bereitzustellen. Insbesondere war es Aufgabe der vorliegenden Erfindung, Substanzen herzustellen, die auch gegen Pilzstämme, bei denen eine weitgehende Resistenz gegenüber N-(3.5-Dichlorphenyl)substituierten Dicarboximiden vorliegt, noch zufriedenstellende Wirksamkeiten zeigen. Des weiteren war es insbesondere die Aufgabe der vorliegenden Erfindung, fungizide Substanzen bereitzustellen, die sowohl gegen Botrytis cinerea, als auch gegen die diesen Pilz häufig begleitenden Pilzarten, wie beispielsweise Altenaria-Arten oder Penicillium-Arten, gute Wirksamkeiten zeigen.

Diese Aufgaben wurden im Rahmen der vorliegenden Erfindung dadurch gelöst, daß Verbindungen der Formel

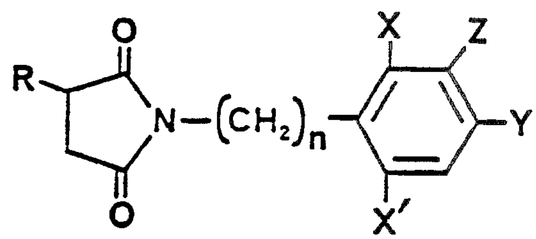

(I)

synthetisiert wurden, worin

R ein Chlor-, Brom- oder Jodatom,

n 0, 1, 2 oder 3,

X, X' und Z jeweils gleiche oder verschiedene Reste, nämlich Wasserstoffatome, Fluoratome oder Methylgruppen, und

Y ein Wasserstoff-, Fluor-, Chlor- oder Bromatom oder eine Methylgruppe bedeuten, mit der Maßgabe, daß für n = 0 und gleichzeitig R = Cl, mindestens einer der Reste X, X', Y und Z kein Wasserstoffatom ist und daß, wenn Z kein Wasserstoffatom bedeutet, mindestens einer der Reste X, X' und Y ebenfalls kein Wasserstoffatom bedeutet.

Bevorzugt sind Verbindungen der Formeln

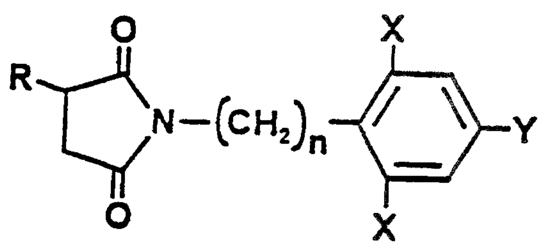

(II)

und

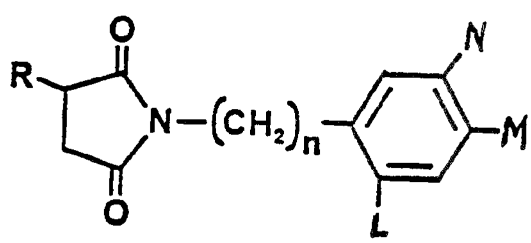

(III)

worin R, n, X, Y die gleichen Bedeutungen wie für die Formel (I) haben, wobei die Reste X jeweils gleiche Reste sind, und

L und N gleiche oder verschiedene Reste, nämlich Methylgruppen oder Fluoratome, und

M ein Wasserstoffatom oder eine Methylgruppe bedeuten.

Besonders bevorzugt aufgrund ihrer fungiziden Wirksamkeit sind diejenigen Verbindungen der Formeln (II) und (III), für die R ein Bromatom bedeutet, insbesondere solche der Formel

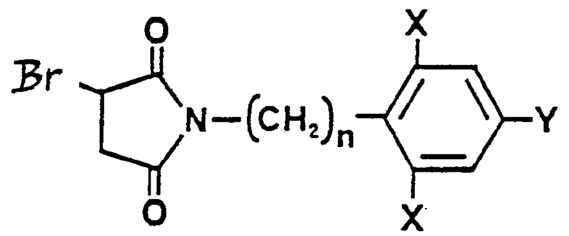

(IV)

worin n, X und Y die für Formel (I) angegebenen Bedeutungen haben, wobei die Reste X jeweils gleiche Reste sind.

Vorzugsweise bedeuten in Formel (IV) die Reste X jeweils zwei Wasserstoffatome oder zwei Methylgruppen.

Wegen ihrer besonders hohen fungiziden Wirksamkeit ist insbesondere-die Verbindung der Formel

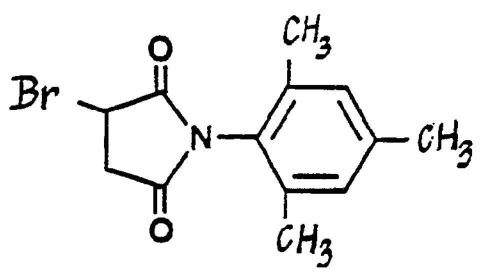

(V)

bevorzugt.

Die erfindungsgemäßen 3-Halogensuccinimide der Formel (I) sind aufgrund eines Asymmetriezentrums am Kohlenstoffatom in 3-Stellung am Heterozyklus optisch aktiv. Es ist durchaus möglich, daß eines der jeweiligen Enantiomeren (3-R- oder 3-S-Enantiomer) stärker als das andere zur gewünschten Wirkung beiträgt. Unter dem Begriff "Verbindungen der Formel" sind auf jeden Fall die optisch reinen 3-R- bzw. 3-S-Enantiomeren sowie jegliche Mischungen von mindestens einem 3-R- mit mindestens einem 3-S-Isomer, beispielsweise die entsprechenden Racemate, zu verstehen

Die erfindungsgemäßen Verbindungen sind beispielsweise wie folgt herstellbar :

1. Durch Umsetzung von Verbindungen der Formel

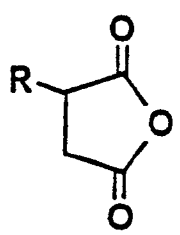

(VI)

mit solchen der Formel

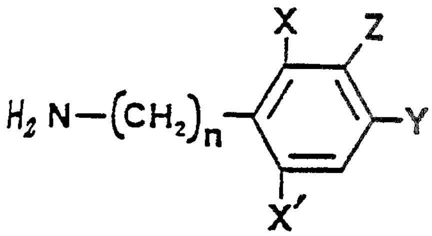

(VII)

zu den entsprechenden Halbamiden und Ringschluß in Gegenwart von die Kondensation fördenden Mitteln, beispielsweise Acetylchlorid ;

2. Durch Umsetzung von Verbindungen der Formel

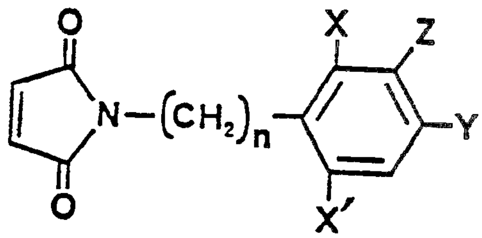

(VIII)

(herstellbar gemäß 1. aus Maleinsäureanhydrid und Verbindungen der Formel (VII)) mit Halogenwasserstoffen der Formel HR in einem geeigneten Lösungsmittel, beispielsweise Trichlormethan.

3. Besonders vorteilhaft ist es, wenn zur Herstellung der erfindungsgemäßen Verbindungen der Formel (I) Verbindungen der Formel

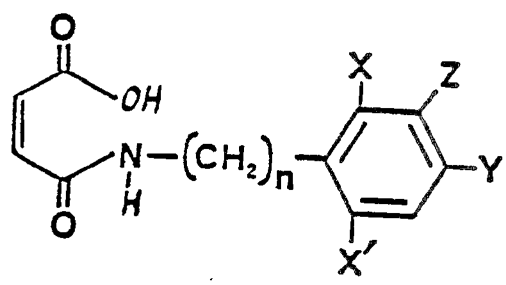

(IX)

mit solchen der Formel

$CH_3COR'$ (X)

umgesetzt werden, wobei R, n, X, X', Y und Z die in Anspruch 1 angegebenen Bedeutungen haben und R' ein Chlor- oder Bromatom bedeutet, und,
falls R ein Jodatom bedeutet, das so erhaltene Produkt der Formel

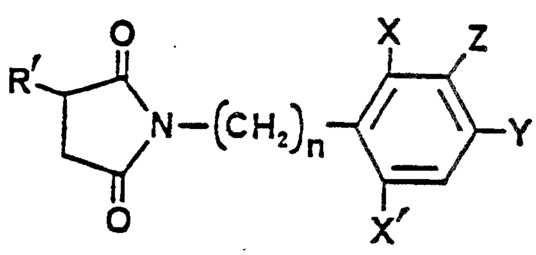

(XI)

anschließend in an sich bekannter Weise mit Alkalimetalljodid, vorzugsweise Natriumjodid, vorzugsweise in einem geeigneten Lösungsmittel, wie Aceton, umgesetzt wird.

Es ist aufgrund der Empfindlichkeit von 3-Jodsuccinimiden gegen Oxidation und Dehydrohalogenierung grundsätzlich von Vorteil, die erfindungsgemäßen 3-Jodsuccinimide in oben geschilderter Weise aus den entsprechenden 3-Chlor- bzw. 3-Bromderivaten herzustellen.

Die Bensteinsäureamide der Formel (IX) sind durch die Reaktion von Verbindungen der Formel (VII) mit Bensteinsäureanhydrid zugänglich, Die als Ausgangsstoffe benötigten Amine der Formel (VII) sind käuflich oder lassen sich in bekannter Weise darstellen.

Nach den vorstehend beschriebenen Methoden wurden die in der folgenden Tab. 1 aufgeführten Verbindungen hergestellt.

Tab. 1

| Wirkst. Nr. | Chemische Bezeichnung | Summenformel | Schmelz. punkt in °C |
|---|---|---|---|
| 1 | 3-Chlor-1-(2.4.6-trimethylphenyl)-2.5-pyrrolidindion | $C_{13}H_{14}ClNO_2$ | 125 |
| 2 | 3-Brom-1-(4-fluorphenyl)-2.5-pyrrolidindion | $C_{10}H_7BrFNO_2$ | 154 |
| 3 | 3-Brom-1-(4-methylphenyl)-2.5-pyrrolidindion | $C_{11}H_{10}BrNO_2$ | 163 |
| 4 | 3-Brom-1-(2.6-difluorphenyl)-2.5-pyrrolidindion | $C_{10}H_6F_2NO_2$ | Öl |
| 5 | 3-Brom-1-(5-fluor-2-methyl-phenyl)-2.5-pyrrolidindion | $C_{11}H_9BrFNO_2$ | 121 |
| 6 | 3-Brom-1-(2-fluor-5-methyl-phenyl)-2.5-pyrrolidindion | $C_{11}H_9BrFNO_2$ | 96 |
| 7 | 3-Brom-1-(2.4.6-trimethyl-phenyl)-2.5-pyrrolidindion | $C_{13}H_{14}BrNO_2$ | 131 |
| 8 | 3-Brom-1-(4-brom-2.6-dimethyl-phenyl)-2.5-pyrrolidindion | $C_{12}H_{11}Br_2NO_2$ | 122 |
| 9 | 3-Brom-1-(2.4-dimethyl-5-fluor-phenyl)-2.5-pyrrolidindion | $C_{10}H_{11}BrFNO_2$ | 115 |
| 10 | 3-Brom-1-(phenylethyl)-2.5-pyrrolidindion | $C_{12}H_{12}BrNO_2$ | 81 |
| 11 | 3-Brom-1-(phenylpropyl)-2.5-pyrrolidindion | $C_{13}H_{14}BrNO_2$ | Öl |
| 12 | 3-Brom-1-(4-methylphenyl)ethyl-2.5-pyrrolidindion | $C_{13}H_{14}BrNO_2$ | 66 |
| 13 | 3-Brom-1-(4-chlorphenyl)ethyl-2.5-pyrrolidindion | $C_{12}H_{11}BrClNO_2$ | 96 |
| 14 | 3-Jod-1-(4-methylphenyl)-2.5-pyrrolidindion | $C_{11}H_{10}JNO_2$ | 159 |
| 15 | 3-Jod-1-(4-fluorphenyl)-2.5-pyrrolidindion | $C_{10}H_7FJNO_2$ | 118 |
| 16 | 3-Jod-1-(2.4.6-trimethylphenyl)-2.5-pyrrolidindion | $C_{13}H_{14}JNO_2$ | 160 |

Die erfindungsgemäßen Verbindungen weisen fungitoxische Eigenschaften auf. Sie werden gegen Pilzbefall an Pflanzen bzw. an pflanzlichen Produkten eingesetzt.

Sie erweisen sich beispielsweise hochwirksam gegen alle Lebensformen von Botrytis cinerea und gegen deren Begleitpilze, wie Alternaria solani und Penicillium glaucum. Es ist insbesondere hervorzuheben, daß die erfindungsgemäßen Wirkstoffe auch gegen solche Botrytis-Stämme wirksam sind, die bereits gegen bekannte Dicarboxanilide, deren gemeinsames Kennzeichen ein an einem Imid-Stickstoff befindlicher 3.S-Dichlorphenyl-Rest ist, Resistenzerscheinungen zeigen.

Ferner werden solche Pilze bekämpft wie Alternaria-Arten, Septoria-Arten, Verticillium dahliae, Colletotrichum-Arten, Monilia-Arten, Fusarium-Arten und Oomyceten, wie z.B. Pythium ultimum.

Die erfindungsgemäßen Wirkstoffe zeichnen sich durch ein breites Wirkungsspektrum aus. So wird beispielsweise nicht nur die Botrytis bekämpft, sondern ebenso die typischen Begleitpilze. Mit den erfindungsgemäßen Wirkstoffen kann mithin der Gesamtkomplex der Pilzkrankheit behandelt werden und damit das bei einseitiger Behandlung oftmals beobachtete vermehrte Aufwachsen von Begleitpilzen verhindert werden.

Die Wirkstoffe eignen sich, ohne daß ihr Anwendungsgebiet darauf beschränkt wäre, z.B. zum Einsatz im Weinbau, im Gartenbau, insbesondere in Salatpflanzungen oder bei Zierpflanzen (Alpenveilchen, Geranien), in Zierrasen, beim Rapsanbau, beim Hopfenanbau, in Erdbeerpflanzungen und im Kernobstbau.

Die Applikation der Wirkstoffe erfolgt in an sich bekannter Weise auf den Lebensraum der Pilze durch beispielsweise Gießen, Verspritzen, Versprühen, Zerstäuben, Bestreichen. Es kann dabei sowohl eine prophylaktische als auch eine kurative Wirkung erzielt werden.

Die erfindungsgemäßen Wirkstoffe können allein oder im Gemisch mit sonstigen Pestiziden, insbesondere fungiziden Mitteln ausgebracht werden. Im allgemeinen werden sie als Mischungen mit festen oder flüssigen Verdünnungsmitteln oder als Lösungen in festen oder flüssigen Lösungsmitteln verwendet, mit Wirkstoffgehalten von 0,005 bis 95 Gew.-%.

Die Mischungen bzw. Lösungen werden im allgemeinen als Emulsionskonzentrate, Pasten, Spritzpulver, Granulate oder Mikrokapseln hergestellt.

Emulsionskonzentrate und Pasten enthalten im allgemeinen 10 bis 90 Gew.-%, vorzugsweise 15 bis 50 Gew.-% Wirkstoff, 2 bis 25 Gew.-% Dispergierhilfsstoffe und organische Lösungsmittel und/oder Wasser.

Spritzpulver enhalten meistens 10 bis 80 Gew.-%, vorzugsweise 15-bis 70 Gew.-% Wirkstoff, 1 bis 30 Gew.-% Dispergierhilfsstoffe und 10 bis 89 Gew.-% inerte Bestandteile.

Granulate und Mikrokapseln enthalten neben inerten Bestandteilen und/oder Oberzugsstoffen 1 bis 10 Gew.-%, vorzugsweise 5 bis 10 Gew.-% Wirkstoff.

Erfindungsgemäß angewandt werden

als Dispergierhilfsstoffe, z.B. Alkyl- und Arylsulfonate, Methylzellulose, polymere Sulfonsäuren und deren Salze, Polyalkohole, Fettsäureester, Fettalkoholether, Fettamine ;

als organische Lösungsmittel, z.B. Alkohole wie Ethanol, Butanole, Dimethylformamid, Dimethylsulfoxid, N-Methyl-Pyrrolidon, Aromaten wie Toluol und Xylole ;

als inerte Bestandteile, z.B. Kaolin, China-Clay, Talkum, Calciumkarbonat, hochdisperse Kieselsäure, Kieselgele, Kieselgur, Diatomeenerde, Bims, Ziegelsplitt, Maisschrot, Verdickungsmittel wie Stärke und Carboxymethylzellulose, Cyclodextrine ;

als Bindemittel, z.B. Magnesiumsulfat, Gips, Gummiarabikum, Polyvinylalkohol.

Beispielsweise werden die erfindungsgemäßen Wirkstoffe zur Verwendung als Fungizide wie folgt formuliert :

<u>Spritzpulver</u>:

| | |
|---|---|
| 20 Gew.-% | Wirkstoff |
| 44 Gew.-% | Chinaclay |
| 16 Gew.-% | hochdisperse Kieselsäure |
| 15 Gew.-% | Ligninsulfonat (Zellpech) |
| 5 Gew.-% | Natrium-Alkylnaphthalinsulfonat-Formaldehyd-kondensat (Atlox 4862, eingetr. Warenzeichen, Hersteller: Atlas-Chemie, D-4300 Essen) |

Emulsionskonzentrat:

| | |
|---|---|
| 20 Gew.-% | Wirkstoff |
| 30 Gew.-% | Cyclohexanon |
| 30 Gew.-% | Xylol |
| 20 Gew.-% | Tween Twenty (eingetragenes Warenzeichen, Hersteller Atlas-Chemie, D-4300 Essen) |

Die folgenden Beispiele dienen zur Erläuterung der Erfindung. Die erfindungsgemäßen Produkte wurden jeweils durch $^1$H-NMR identifiziert

Beispiel 1 : Herstellungsbeispiel

3-Brom-1-(4-fluorphenyl)-2.5-pyrrolidindion, Wirkstoff 2

29,4 g (0.3 Mol) Maleinsäureanhydrid wurden in 300 ml Toluol gelöst und dazu bei 30°C eine lösung von 33,3 g (0.3 Mol) 4-Fluoranilin in 100 ml Toluol zugegeben. Das Halbanilid fiel rasch aus. Zur Vervollständigung der Reaktion wurde das Gemisch 2 Stunden auf 100°C erwärmt, dann abgekühl ;, das ausfallende Produkt abfiltriert, gewaschen und getrocknet. Die Ausbeute an Halbanilid war quantitativ. Das Halbanilid wurde in Essigsäureethylester suspendiert und dazu 43 g (0.35 Mol) Acetylbromid zugegeben. Durch Rühren bei 30°C (2 Std.) und durch nachfolgendes 2-stündiges Erhitzen auf Rückfluß wurde die Reaktion durchgeführt, danach das Lösungsmittel abdestilliert und der Rest kristallisiert. Die Ausbeute betrug 69,2 g (entsprechend 85,5% d.Th.) Das Produkt wies einen Schmelzpunkt von 154°C auf. NMR (in $CDCl_3$ als Lösungsmittel, Tetramethylsilan als innerer Standard) : 2,9-3,8 ppm (4 Dubletts, J = 3 bzw. 9 Hz ; Ring-$CH_2$), 4,8 bzw. 4,95 (2 Dubletts, J = 3 Hz ; Ring-CH) 7,0-7,45 (Multiplett, 4 aromat. Protonen) im Verhältnis 2 : 1 : 4.

Beispiel 2, Herstellungsbeispiel

3-Jod-1-(4-fluorphenyl)-2.5-pyrrolidindion, Wirkstoff 15

27,2 g (0.1 Mol) des nach Beispiel 1 hergestellten 3-Brom-1-(4-fluorphenyl)-2.5-pyrrolidindions wurden in 140 ml Aceton gelöst und dazu eine lösung von 15,75 (0.105 Mol) Natriumjodid in Aceton zugegeben. Das Gemisch wurde 2 Stunden bei Zimmertemperatur gerührt und dann 1 Stunde auf Rückfluß erhitzt. Anschließend wurde abgekühlt, vom entstandenen Natriumbromid abfiltriert und das Filtrat eingeengt. Der Rückstand wurde aus Methanol/Wasser (3 : 1) umkristallisiert und ergab das gesuchte Produkt mit 70,5% d.Th. Der Schmelzpunkt des Derivats liegt bei 118°C.

Beispiel 3, Herstellungsbeispiel

3-Brom-1-(2.4-dimethyl-5-fluorphenyl)-2.5-pyrrolidindion, Wirkstoff 9

13,9 g (0.1 Mol) 2.4-Dimethyl-5-fluoranilin und 9,8 g (0.1 Mol) Maleinsäureanhydrid wurden, wie in Beispiel 1 beschrieben, miteinander umgesetzt. Die Ausbeute betrug 70,2% d.Th ; der Schmelzpunkt des Produkts liegt bei 115°C.

Herstellung des Ausgangsmaterials (nach B.C. Becker und R. Adams, J. Am. chem. Soc. 54, 2980 (1932)): 60,5 g käufliches 2.4-Dimethylanilin wurden langsam bei 0-10°C zu 340 ml konz. Schwefelsäure gegeben und dazu, ebenfalls bei 0-10°C 72,5 ml konz. Salpetersäure (D = 1,4) im Verlauf einer Stunde unter Rühren zugetropft. Anschließend wurde noch 30 min. weitergerührt, dann das Gemisch auf Eis gegossen und der Niederschlag mit 35 g Natriumnitrit bei 0-10°C in etwa 2,5 Std. diazotiert. Nach weiteren 30 min Rühren wurden bei 0°C 88 ml 50% wässr. $HBF_4$ zugetropft, wieder 30 min. gerührt und dann der ausgefallene Niederschlag abfiltriert (52,5 g nach Waschen und Trocknen). Dieses Produkt wurde nun portionsweise im Verlauf von 5 Std. bei 130°C zersetzt und destilliert ($Kp_{12}$ 103°C). Die Ausbeute betrug 14,8 g (entspricht 17,4% d.Th.).

Diese Nitroverbindung wurde nun in 125 ml Methanol gelöst und mit $PtO_2$ als Katalysator mit Wasserstoff bei 35-45°C (exotherme Reaktion) im Verlauf von 3 Std. hydriert. Durch Filtrieren, Einengen und Kristallisieren

wurden 11 g (entsprechend 93% d.Th.) des gesuchten Anilinderivats erhalten. Schmelzpunkt des Amins 58-60°C.

Beispiel 4, Herstellungsbeispiel

3-Brom-1-(4-chlorphenyl)ethyl-2.5-pyrrolidindion, Wirkstoff 13

9,8 g (0.1 Mol) Maleinsäureanhydrid wurden in 100 ml Toluol gelöst und dazu bei 30°C eine lösung von 15,6 g (0.1 Mol) käuflichem 2-(4-Chlorphenyl)ethylamin in Toluol zugegeben. Das Maleinsäurehalbamid fällt rasch aus. Nach weiteren zwei Stunden wurde das Halbamid abfiltriert (Fp 136-138°C). Anschließend wurde es in Essigsäureethylester suspendiert, 12,9 g (0.105 Mol) Acetylbromid zugefügt und das Gemisch unter Rühren 2 h auf 30-40°C erwärmt, wobei der Niederschlag allmählich aufgelöst wurde. Danach wurde weitere 2 Stunden auf Rückfluß erhitzt, dann das Lösungsmittel abgezogen und der Rückstand kristallisiert. Das Produkt kann aus Ethanol umkristallisiert werden und schmilzt dann bei 96°C. Die Ausbeute beträgt 78,0% d.Th.

Für die folgenden Wirkungsbeispiele wurden aus dem bekannten Stand der Technik drei besonders nahe liegende Substanzen als Vergleichsmittel ausgewählt. Es sind dies :

1. 3-Brom-1-(3.5-dichlorphenyl)-2.5-pyrrolidindion, bekannt aus DE 2.012.656, im folgenden als "Vergleich A" bezeichnet,
2. 3-Chlor-1-(2.4.6-trimethylphenyl)-1H-pyrrol-2.5-dion, bekannt aus FR 2.226.396, im folgenden als "Vergleich B" bezeichnet,
3. 3-Chlormethyl-1-(2.4.6-trimethylphenyl)-2.5-pyrrolidindion, bekannt aus EPA 0.173.284, im folgenden als "Vergleich C" bezeichnet.

Beispiel 5, Wirkungsbeispiel

Sporenkeimtest

50 µl einer Lösung oder Suspension eines Wirkstoffs mit einem Gehalt von 250 bzw. 16 ppm Aktivsubstanz wurden zusammen mit 50 µl einer Sporensuspension, hergestellt durch Abschwemmen der Sporen von einer Agarkultur mit einer Nährlösung, die pro Liter 10 g Zucker, 1 g Glykol, 1 g $KH_2PO_4$ und 0,5 g $MgSO_4$ enthielt, in den Hohlschliff von Hohlschliffobjektträgern eingebracht. Die Objektträger wurden bei 20°C 48 Stunden in einer Petrischale, deren Boden mit angefeuchtetem Filterpapier bedeckt war, aufbewahrt.

Danach wurde das Verhältnis der gekeimten und der nicht gekeimten Sporen gegen eine unbehandelte Kontrollprobe verglichen.

Der Wirkungsgrad wird in % nach der folgenden Formel angegeben :

$$100 - \frac{\text{Anzahl der gekeimten Sporen, behandelt}}{\text{Anzahl der gekeimten Sporen, unbehandelt}} \times 100$$

Die Ergebnisse sind in den folgenden Tab. 2 und 3 zusammengestellt.

Tab . 2: Fungitoxizität erfindungsgemäßer 2.5-Pyrrolidindione bei 250 ppm Wirkstoffkonzentration in %

| Wirkstoff | Alternaria solani | Botrytis cinerea | Fusarium culmorum | Fusarium nivale | Colletotrichum coffeanum | Verticillium dahliae | Penicillium glaucum |
|---|---|---|---|---|---|---|---|
| 1 | 100 | 80 | 100 | 100 | 100 | 100 | 80 |
| 2 | 100 | 100 | 100 | 100 | 100 | 100 | 70 |
| 3 | 40 | 100 | 100 | 100 | 100 | 100 | 60 |
| 4 | 100 | 100 | 100 | 100 | 100 | 100 | 100 |
| 5 | 100 | 100 | 100 | 100 | 100 | 100 | 100 |
| 6 | 100 | 100 | 100 | 100 | 100 | 100 | 100 |
| 7 | 100 | 100 | 100 | 100 | 100 | 100 | 100 |
| 8 | 100 | 100 | 100 | 100 | 100 | 100 | 100 |
| 9 | 100 | 100 | 95 | 100 | 100 | 100 | 95 |
| 10 | 100 | 100 | 100 | 100 | 100 | 100 | 100 |
| 11 | 100 | 80 | 100 | 100 | 100 | 100 | 100 |
| 12 | 100 | 100 | 90 | 100 | 100 | 100 | 100 |
| 13 | 100 | 100 | 100 | 100 | 100 | 100 | 10 |
| 14 | 80 | 100 | 100 | 100 | 100 | 100 | 0 |
| 15 | 100 | 100 | 100 | 100 | 100 | 100 | 100 |
| 16 | 100 | 100 | 100 | 100 | 100 | 100 | 100 |

Tab. 3: Fungitoxizität erfindungsgemäßer 2.5-Pyrrolidindione und von Vergleichssubstanzen bei 16 ppm Wirkstoffkonzentration

| Wirkstoff | Alternaria solani | Botrytis cinerea | Fusarium culmorum | Fusarium nivale | Colletorichum coffeanum | Verticillium dahliae | Penicillium glaucum |
|---|---|---|---|---|---|---|---|
| 1 | 100 | 30 | 20 | 100 | 100 | 80 | 0 |
| 3 | 30 | 100 | 40 | 100 | 100 | 80 | 0 |
| 7 | 100 | 80 | 90 | 100 | 100 | 100 | 80 |
| 8 | 100 | 80 | 100 | 100 | 100 | 100 | 75 |
| 13 | 100 | 85 | 70 | 100 | 100 | 100 | 10 |
| 14 | 60 | 70 | 80 | 100 | 100 | 100 | 0 |
| A | 0 | 100 | 30 | 80 | 90 | 80 | 30 |
| B | 20 | 0 | 0 | 0 | 10 | 0 | 0 |
| C | 25 | 10 | 0 | 90 | 40 | 35 | 0 |

Beispiel 6, Wirkungsbeispiel

Traubensafttest

20 ml eine Nährlösung aus Traubensaft und destilliertem Wasser im Verhältnis 1 : 1 wurden in Petrischalen eingefüllt und mit den in der Folgenden Tabelle angeführten Wirstoffen versetzt. Die Wirkstoffkonzentration betrug 31 ppm. Anschließend wurden die Versuchsansätze mit jeweils 50 µl einer Botrytis-Sporensuspension, hergestellt durch Abschwemmen der Botrytis-Sporen von einer Agarkultur mit destilliertem Wasser, beimpft.

Nach einer Bebrütungsdauer von 10 bzw. 20 Tagen bei 20°C wurde das Ausmaß der Pilzentwicklung auf der Nährlösungsoberfläche beurteilt.

Der Wirkungsgrad wurde in % nach der folgenden Formel errechnet:

$$100 - \frac{\text{Pilzwachstum, behandelt}}{\text{Pilzwachstum, unbehandelt}} \times 100$$

Tab. 4:

Wirksamkeit von erfindungsgemäßen Stoffen und von Vergleichsmitteln in %-Graden bei 31 ppm Wirkstoffkonzentration nach 10 bzw. 20 Tagen Einwirkungs- dauer

| Wirkstoff Nr. | % Wirksamkeit nach 10 Tagen | nach 20 Tagen |
|---|---|---|
| 1 | 100 | 100 |
| 3 | 100 | 100 |
| 4 | 100 | 100 |
| 5 | 100 | 85 |

| Wirkstoff Nr. | % Wirksamkeit nach 10 Tagen | nach 20 Tagen |
|---|---|---|
| 6 | 100 | 90 |
| 7 | 100 | 100 |
| 8 | 100 | 100 |
| 9 | 100 | 100 |
| 11 | 100 | 90 |
| 12 | 100 | 100 |
| 13 | 100 | 100 |
| 14 | 100 | 100 |
| 15 | 100 | 100 |
| 16 | 100 | 100 |
| A | 100 | 100 |
| B | 95 | 70 |
| C | 60 | 20 |

Besonders aktive erfindungsgemäße Wirkstoffe, wie z.B. Wirkstoff 16, zeigen selbst bei 2 ppm noch 80% Wirksamkeit nach 20 Tagen.

Beispeil 7, Wirkungsbeispiel

Traubensafttest mit resistenten Botrytis cinerea-Stämmen

Die Arbeitsweise nach Beispiel 6 wurde mit der Abänderung wiederholt, daß mit Botrytissporen eines Botrytisstammes beimpft wurde, der gegen N-(1.5-Dichlorphenyl)-substituierte Imide Resistenz zeigt. Die Wirkstoffkonzentration betrug 8 ppm. Während mit Wirkstoff 7 gegen diesen Stamm eine 65%ige Wirksamkeit erreicht wurde, war mit Vergleich A bei gleicher Wirkstoffkonzentration nur 20% Wirksamkeit festzustellen. Als zusätzlicher Vergleich wurde "Ronilan" (= 3-(3.5-Dichlorphenyl)-5-ethenyl-5-methyl-2.4-oxazolidindion ; eingetragener Markenname der BASF AG) verwendet, das gegen den gleichen Stamm selbst bei 62 ppm Wirkstoffkonzentration nur 26% Wirksamkeit erzielte.

Beispiel 8, Wirkungsbeispiel

Botrytis cinerea an Paprika (Capsicum annuum)

Junge Paprikapflanzen, die das erste echte Blattpaar entwickelt haben, werden mit einer wässrigen Lösung der Wirkstofformulierung tropfnaß gespritzt. Nach dem Antrocknen des Spritzbelags werden die Pflanzen mit einer Suspension von Botrytissporen künstlich infiziert und danach bei 95-98% Luftfeuchtigkeit und 20°C in einer Klimakammer aufbewahrt. 6 Tage nach der Inokulation wird der Versuch bonitiert. Die Befallsgrade werden durch Vergleich zu unbehandelten Kontrollpflanzen sowie einem Vergleichspräparat ermittelt.

Tab. 5 :

Wirksamkeit von erfindungsgemäßen Wirkstoffen und von Vergleichsmitteln in %-Graden bei 500 ppm Wirkstoffkonzentration an Paprikapflanzen.

| Wirkstoff Nr. | % Wirksamkeit |
|---|---|
| 2 | 80 |
| 3 | 90 |
| 7 | 85 |
| 14 | 95 |
| A | 45 |
| B | 50 |
| C | 10 |

Führt man den gleichen Versuch mit Botrytis-Sporen durch, die wie in Beispiel 7 gegen N-(3.5-Dichlorphenyl)substituierte Imide Resistenzerscheinungen zeigen, so sinkt der Wirksamkeitsgrad beispielsweise mit Vergleichssubstanz A auf etwa die Hälfte des Wertes mit sensiblen Stämmen ab, während beispielsweise mit Wirkstoff 7 praktisch die gleiche Wirksamkeit gegen sensible wie auch gegen resistente Stämme erhalten wurde.

Beispiel 9, Wirkungsbeispiel

Wirksamkeit gegen Puccinia triticina (Weizenbraunrost)

Junge Weizenpflanzen im 2-Blatt-Stadium wurden mit einer wässrigen Wirkstoffsuspension tropfnaß gespritzt. Nach dem Antrocknen des Wirkstoffbelags wurden die Pflanzen durch Besprühen mit einer wässrigen Uredo-Sporen-Suspension infiziert. Nach 24-stündigem Stehen in einer dunklen Feuchtkammer wurden die Pflanzen im Gewächshaus bei 20 bis 22°C und 65 bis 70% rel. Luftfeuchte aufgestellt. Nach 10 bis 14 Tagen Inkubationszeit wurden die Prozentzahlen der Befallsgrade durch Vergleich der befallenen Blattflächen mit unbehandelten, infizierten Kontrollpflanzen ermittelt.

Tab. 6 :

Wirksamkeit von erfindungsgemäßen Verbindungen und von Vergleichsmitteln in %-Graden bei 500 ppm Wirkstoffkonzentration

| Wirkstoff Nr. bzw. Vergleich | % Wirksamkeit |
|---|---|
| 1 | 90 |
| 7 | 90 |
| 14 | 70 |
| 15 | 80 |
| 16 | 100 |
| A | 0 |
| B | 65 |
| C | 50 |

Gegen Bohnenrost (Uromyces phaseoli) werden meist ganz ähnliche Bekämpfungswerte erzielt wie gegen Weizenbraunrost.

Beispeil 10, Wirkungsbeispiel

Wirksamkeit gegen Pythium ultimum bei Bodenapplikation

Der Wirkstoff wurde in einer Konzentration von 500 ppm gleichmäßig in Erde gemischt, die mit Pythium ultimum künstlich infiziert war. Die so behandelte Erde wurde in Plastiktöpfe gefüllt (je 4 Wiederholungen pro Testsubstanz) und mit je 10 Erbsensamen besät. Diese Töpfe wurden 10 Tage bei 24 bis 26°C und einer Luftfeuchte von 75 bis 90% aufbewahrt. Danach wurde die Anzahl der gesunden, aufgelaufenen Pflanzen bestimmt. Der Wirkungsgrad wurde durch Vergleich mit infizierten, aber unbehandelten Erdproben errechnet. Die Ergebnisse sind in der nachfolgenden Tabelle zusammengestellt.

Tab. 7 :

Wirksamkeit von erfindungsgemäßen Wirkstoffen und von Vergleichsmitteln in % Graden bei 500 ppm Wirkstoffkonzentration gegen Pythium ultimum.

| Wirkstoff Nr. | % Wirksamkeit |
|---|---|
| 1 | 100 |
| 7 | 100 |
| 8 | 100 |
| 11 | 75 |
| 12 | 75 |
| 16 | 80 |
| A | 25 |
| B | 0 |
| C | 45 |

**Ansprüche**

1. Verbindungen der Formel

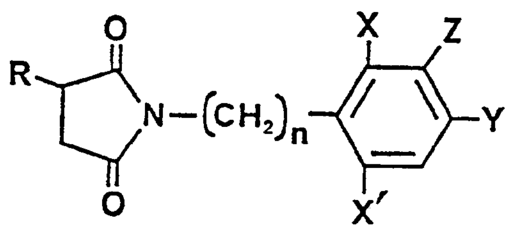

(I)

worin

R ein Chlor-, Brom- oder Jodatom,

n 0, 1, 2 oder 3,

X, X' und Z jeweils gleiche oder verschiedene Reste, nämlich Wasserstoffatome, Fluoratome oder Methylgruppen, und

Y ein Wasserstoff-, Fluor-, Chlor- oder Bromatom oder eine Methylgruppe bedeuten, mit der Maßgabe, daß für n = 0 und gleichzeitig R = Cl, mindestens einer der Reste X, X', Y und Z kein Wasserstoffatom ist und daß, wenn Z kein Wasserstoffatom bedeutet, mindestens einer der Reste X, X' und Y ebenfalls kein Wasserstoffatom bedeutet.

2. Verbindungen nach Anspruch 1, **dadurch gekennzeichnet**, daß sie die Formeln

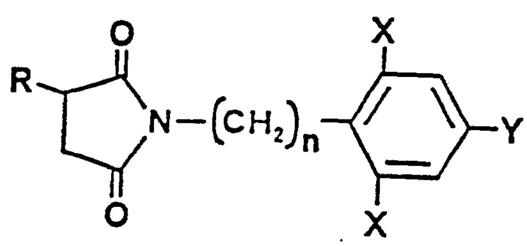

(II)

und

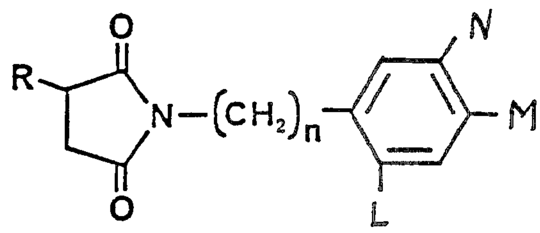

(III)

aufweisen,

worin R, n, X, Y die gleichen Bedeutungen wie für die Formel (I) haben, wobei die Reste X jeweils gleiche Reste sind, und

L und N gleiche oder verschiedene Reste, nämlich Methylgruppen oder Fluoratome, und

M ein Wasserstoffatom oder eine Methylgruppe bedeuten.

3. Verbindungen nach Anspruch 1, **dadurch gekennzeichnet**, daß sie die Formel

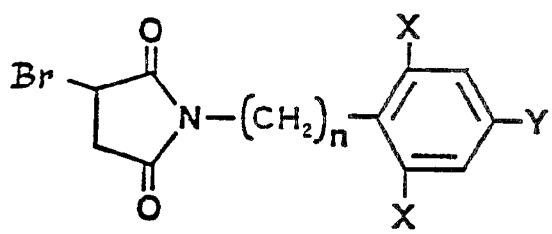

(IV)

aufweist

worin n, X und Y die für Formel (I) angegebenen Bedeutungen haben, wobei die Reste X jeweils gleiche Reste sind, aufweisen.

4. Verbindung nach Anspruch 1, **dadurch gekennzeichnet**, daß sie die Formel

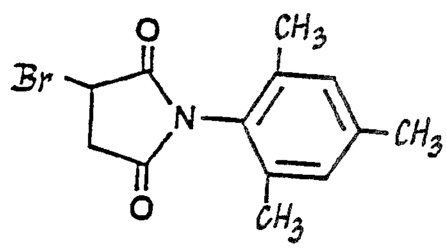

(V)

aufweist.

5. Verfahren zur Herstellung von Verbindungen der Formel

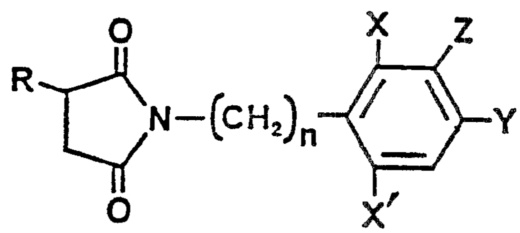

(I)

worin R, n, X, X′, Y und Z die in Anspruch 1 angegebenen Bedeutungen haben, **dadurch gekennzeichnet**, daß Verbindungen der Formel

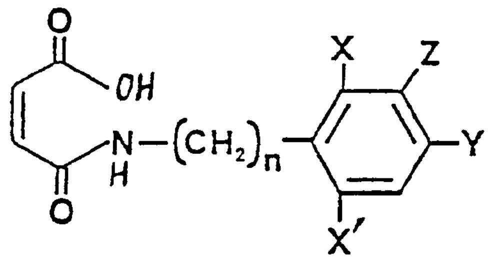

(IX)

mit solchen der Formel

$CH_3COR'$ (X)

umgesetzt werden, wobei R, n, X, X′, Y und Z die in Anspruch 1 angegebenen Bedeutungen haben und R′ ein Chlor- oder Bromatom bedeuten, wobei dann, wenn n gleich 0 und R gleich Cl ist, mindestens einer der Rest X, X′, Y und Z kein Wasserstoffatom ist, und falls R in der Formel (I) ein Jodatom bedeutet, das so erhaltene Produkt der Formel

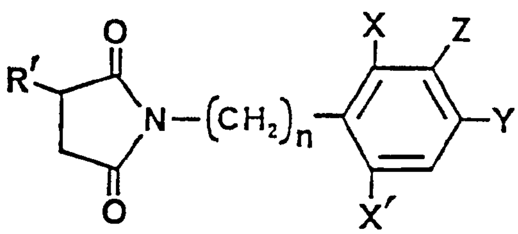

(XI)

anschließend in an sich bekannter Weise mit Alkalimetalljodid umgesetzt wird.

6. Verwendung der Verbindungen gemäß Anspruch 1, 2, 3 oder 4 als fungizide Wirkstoffe.

7. Verwendung der gemäß Anspruch 5 hergestellten Verbindungen als fungizide Wirkstoffe.

**Claims**

1. Compounds of formula

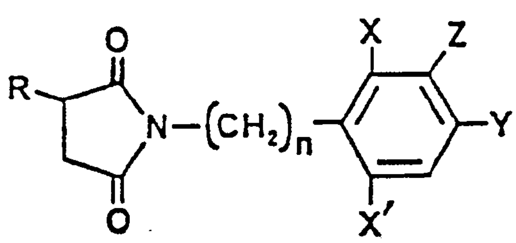

(I)

in which R is a chlorine, bromine or iodine atom, n is 0, 1, 2 or 3, X, X′ and Z, are identical or different radicals, namely hydrogen atoms, fluorine atoms or methyl groups and Y stands for a hydrogen, fluorine, fluorine or bromine atom or a methyl group, provided that for n = 0 and at the same time R = Cl, at least one of the radicals X, X′, Y and Z is not a hydrogen atom and that if Z is not a hydrogen atom at least one of the radicals X, X′ and Y is also not a hydrogen atom.

2. Compounds according to claim 1, characterized in that they have the formulae

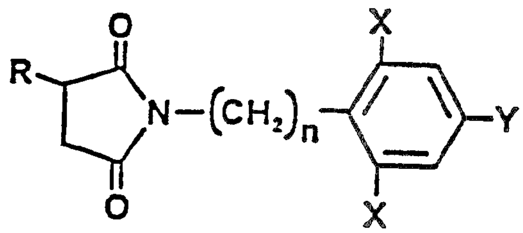

(II)

and

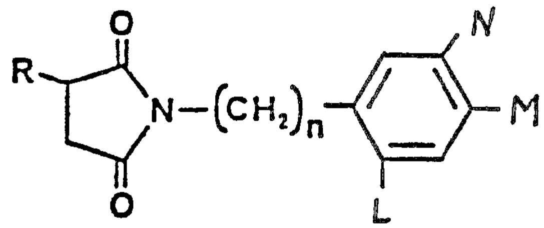

(III)

in which R, n, X and Y have the same meanings as for formula (I), in which the radicals X are in each case identical radicals and L and N stand for identical or different radicals, namely methyl groups or fluorine atoms and M stands for a hydrogen atom or a methyl group.

3. Compounds according to claim 1, characterized in that they have the formula

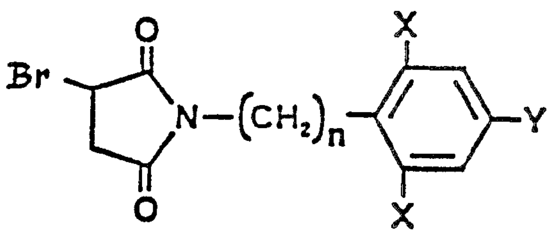

(IV)

in which n, X and Y have the meanings given for formula (I) and in which the radicals X are in each case identical radicals.

4. Compound according to claim 1, characterized in that they have the formula

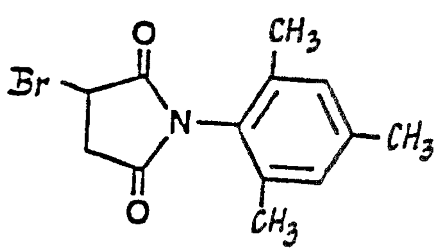

(V)

5. Process for the preparation of compounds of formula

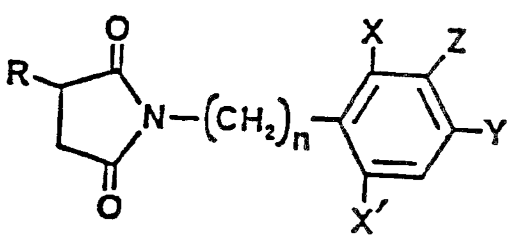

(I)

in which R, n, X, X′, Y and Z have the meanings given in claim 1, characterized in that compounds of formula

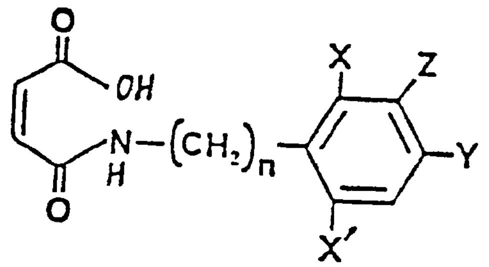

(IX)

are reacted with those of the formula

$CH_3COR'$ (X)

in which R, n, X, X′, Y and Z have the meanings given in claim 1 and R′ stands for a chlorine or bromine atom and in which if n is equal to 0 and R is equal to Cl, at least one of the radicals X, X′, Y and Z is not a hydrogen atom and that if R in formula (I) stands for an iodine atom, the thus obtained product of formula

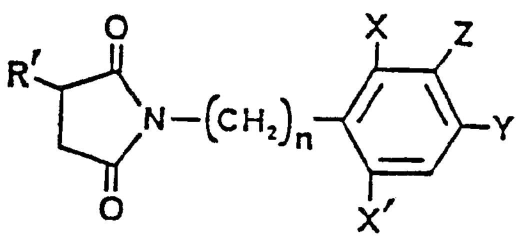

(XI)

is subsequently reacted in per se known manner with alkali metal iodide.

6. Use of the compounds according to claims 1, 2, 3 or 4 as fungicidal active substances.

7. Use of the compounds prepared according to claim 5 as fungicidal active substances.

**Revendications**

1. Composés de formule

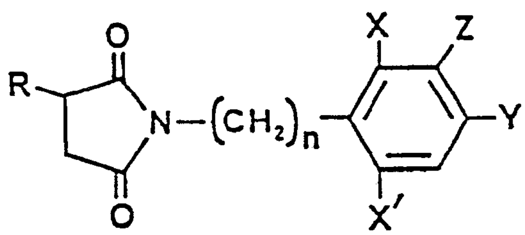

(I)

dans laquelle

R représente un atome de chlore, de brome ou d'iode,

n vaut 0, 1, 2 ou 3,

X, X′ et Z sont des restes identiques ou différents, et représentent chacun un atome d'hydrogène, un atome de fluor ou un groupe méthyle, et

Y représente un atome d'hydrogène, de fluor, de chlore ou de brome, ou un groupe méthyle, étant entendu que, pour n = 0 et en même temps R = Cl, au moins l'un des restes X, X′, Y et Z ne représente pas un atome d'hydrogène, et que, lorsque Z ne représente pas un atome d'hydrogène, au moins l'un des restes X, X′, Y ne représente pas non plus un atome d'hydrogène.

2. Composés selon la revendication 1, caractérisés en ce qu'ils répondent à la formule

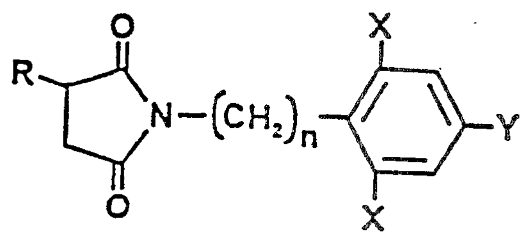

(II)

ou à la formule

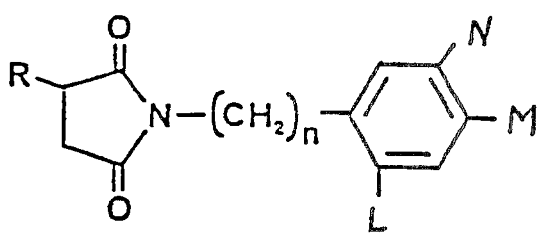

(III)

dans lesquelles R, n, X, Y ont les mêmes significations que dans la formule (I), les restes X étant toujours identiques, et

L et N sont des restes identiques ou différents, et représentent chacun un groupe méthyle ou un atome de fluor, et

M représente un atome d'hydrogène ou un groupe méthyle.

3. Composés selon la revendication 1, caractérisés en ce qu'ils répondent à la formule

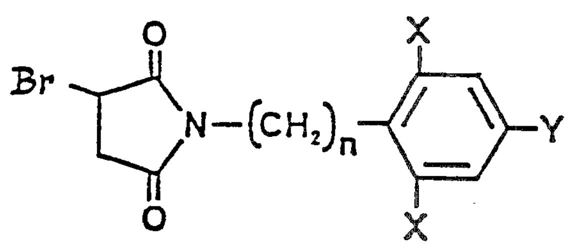

(IV)

dans laquelle

n, X et Y ont les significations mentionnées pour la formule (I), les restes X étant toujours identiques.

4. Composé selon la revendication 1, caractérisé en ce qu'il répond à la formule

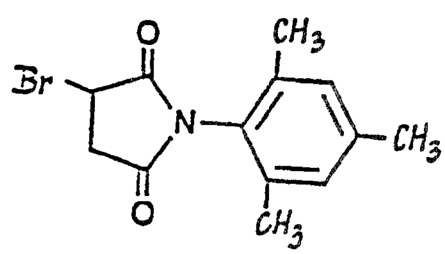

(V)

5. Procédé pour la préparation de composés de formule

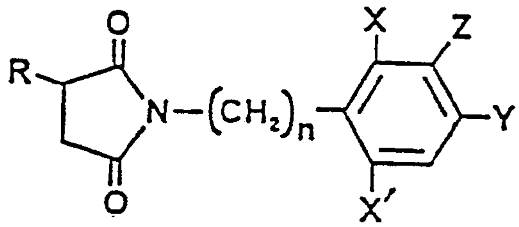

(I)

dans laquelle R, n, X, X', Y et Z ont les significations mentionnées à la revendication 1, caractérisé en ce que l'on fait réagir les composés de formule

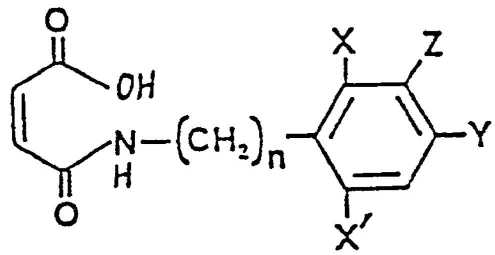

(IX)

avec des composés de formule

$CH_3COR'$ (X)

formules dans lesquelles R, n, X, X', Y et Z ont les significations mentionnées à la revendication 1, et R' représente un atome de chlore ou de brome, étant entendu que lorsque n vaut 0 et R représente Cl, au moins l'un des restes X, X', Y et Z est différent de l'hydrogène, et en ce que,
lorsque R dans la formule (I) représente un atome d'iode, le produit ainsi obtenu, répondant à la formule

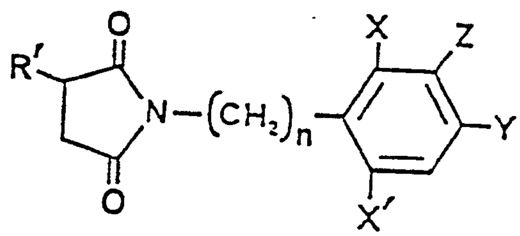

(XI)

est mis ensuite à réagir de manière connue en soi avec un iodure alcalin

6. Utilisation des composés selon les revendications 1, 2, 3 ou 4 comme agents actifs fongicides.

7. Utilisation des composés préparés selon la revendication 5, comme agents actifs fongicides.